# EUROPEAN PATENT APPLICATION

(11) **EP 3 524 149 A2**
(43) Date of publication of application: **14.08.2019**
(21) Application number: 19164916.9
(22) Date of filing: 04.11.2014
(51) Int. Cl.: A61B 5/06, A61B 5/00

(54) **ELECTRICALLY TRANSPARENT CATHETER SHEATH**

(30) Priority: 05.11.2013 US 201314071711
(62) Divisional of application: 14191696.5
(71) Applicant: Biosense Webster (Israel) Ltd., Yokneam, 2066717 (IL)
(72) Inventor: LICHTENSTEIN, Yoav, 4372706 Raanana (IL)
(74) Representative: Small, Gary James

(57) **Abstract**

A sheath includes an elongate tube, which has an outer wall that surrounds an inner lumen and has multiple holes along the tube penetrating the outer wall. Multiple electrically-conducting elements are inserted in the respective holes, so as to allow transmission of electrical current between the inner lumen and an exterior of the outer wall.

## Description

### FIELD OF THE INVENTION

The present invention relates generally to catheter sheaths, and particularly to catheter location tracking in sheaths.

### BACKGROUND OF THE INVENTION

A wide range of medical procedures involve inserting sensors, catheters, implants, and other medical devices in the human body and guiding them to a particular position within the body. When a catheter is inserted into the body, impedance-based position sensing is one method used to guide the tip of the catheter to the target position in which the medical procedure is to be performed. In some procedures, catheters are guided in the human body within the lumen of a sheath.

U.S. Patent 7,869,865, whose disclosure is incorporated herein by reference, describes a method for position sensing which includes inserting a probe comprising at least one electrode into a body of a subject, and passing electrical currents through the body between the at least one electrode and a plurality of locations on a surface of the body. Respective characteristics of the currents passing through the plurality of the locations are measured in order to determine position coordinates of the probe.

U.S. Patent 6,582,536, whose disclosure is incorporated herein by reference, describes a method for manufacturing a steerable catheter having a distal end, proximal end, an outer jacket, a pull wire and a central lumen. The central lumen is maintained in a circular shape without bulges diminishing the useful inter-diameter by using an outer jacket with an elliptical shape and uneven thickness to encase a pull wire.

### SUMMARY OF THE INVENTION

An embodiment of the present invention that is described herein provides a sheath including an elongate tube, which has an outer wall that surrounds an inner lumen and has multiple holes along the tube penetrating the outer wall. Multiple electrically-conducting elements are inserted in the respective holes, so as to allow transmission of electrical current between the inner lumen and an exterior of the outer wall.

In some embodiments, the electrically-conducting elements include metallic beads filling the multiple holes. In other embodiments, the electrically-conducting elements include metallic rivets filling the multiple holes. In yet other embodiments, the electrically-conducting elements include metallic springs threaded through the multiple holes. In some embodiments, the elongate tube includes metallic filaments forming a braid, and the sheath includes an insulating material that insulates the elements from the metallic filaments of the braid.

There is also provided, in accordance with an embodiment of the present invention, a method including producing an elongate tube, which includes an outer wall that surrounds an inner lumen and has multiple holes along the tube penetrating the outer wall. Multiple electrically-conducting elements are inserted into the respective holes so as to allow transmission of electrical current between the inner lumen and an exterior of the outer wall.

In other embodiments, producing the tube and inserting the electrically-conducting elements include mixing metallic objects in an insulating material used for producing the tube, and then forming the tube by extrusion. In yet other embodiments, producing the tube includes drawing insulating material over a mold having multiple openings, and inserting the electrically-conducting elements includes injecting metal through the openings.

There is also provided, in accordance with an embodiment of the present invention, an apparatus including a plurality of body surface electrodes, a sheath, a probe, and a processor. The body surface electrodes are fixed at respective locations on a surface of a living body. The sheath is inserted into the living body and includes an elongate tube. The elongate tube includes an outer wall that surrounds an inner lumen and has multiple holes along the tube penetrating the outer wall. Multiple electrically-conducting elements are inserted in the respective holes so as to allow transmission of electrical current between the inner lumen and an exterior of the outer wall. The probe includes at least one probe electrode, and is guided through the inner lumen of the sheath. The processor is configured to measure the electrical current that flows via the elements between the at least one probe electrode and the body surface electrodes, and to estimate a position of the at least one probe electrode based on the measured electrical current.

There is also provided, in accordance with an embodiment of the present invention, a method including fixing a plurality of body surface electrodes at respective locations on a surface of a living body. A sheath inserted into the living body includes an elongate tube. The elongate tube includes an outer wall that surrounds an inner lumen and has multiple holes along the tube penetrating the outer wall. Multiple electrically-conducting elements are inserted in the respective holes so as to allow transmission of electrical current between the inner lumen and an exterior of the outer wall. A probe including at least one probe electrode is guided through the inner lumen of the sheath. The electrical current that flows via the elements is measured between the at least one probe electrode and the body surface electrodes. A position of the at least one probe electrode is estimated based on the measured electrical current.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a position sensing system, in accordance with an embodiment of the present invention;
Fig. 2 is a schematic, pictorial illustration of a sheath with metal-filled holes, in accordance with an embodiment of the present invention;
Fig. 3 is a schematic, pictorial illustration of a sheath with holes filled with metal rivets, in accordance with an embodiment of the present invention;
Fig. 4 is a schematic, pictorial illustration of a sheath with holes filled with metal springs, in accordance with an embodiment of the present invention; and
Fig. 5 is a flow chart that schematically illustrates a method for sensing the position of a catheter in an electrically-transparent sheath, in accordance with an embodiment of the present invention.

### DETAILED DESCRIPTION OF EMBODIMENTS

### OVERVIEW

Medical probes, such as catheters, are used in a variety of therapeutic and diagnostic procedures. Typically, a catheter is inserted percutaneously into the patient, and an operator guides the catheter to a target region in the body cavity where the procedure is to be performed. In some procedures, the operator first inserts a sheath into the patient body and guides the sheath's distal end to the target region. The operator then inserts the catheter into the lumen of the sheath and guides the catheter distal tip through the sheath to the target region.

A position sensing system may be used for detecting the position of the catheter's distal tip as the operator manipulates the catheter in the patient's body cavity, for example in a chamber of the heart. In impedance-based position sensing systems, for example, the system generates and then measures a plurality of currents between at least one electrode disposed near the catheter distal tip and a plurality of body surface electrodes, fixed at respective locations on a surface of the patient body. The system then computes a plurality of impedances based on the measured currents and detects the position of the catheter's distal tip using the computed impedances.

If, however, the catheter is guided via an electrically-insulating sheath, the position of the catheter's distal tip cannot be measured correctly using impedance-based location sensing while the tip is inside the sheath. Since the insulating sheath prevents current from flowing between the catheter and the body surface electrodes, the system is essentially "blind" to the catheter's position within the insulating sheath until the catheter's distal tip emerges from the sheath close to the target region.

Embodiments of the present invention that are described herein provide sheathes and associated methods which enable impedance-based position sensing systems to detect the position of the catheter's distal tip even while the catheter is within the lumen of the sheath.

In order to make the sheath suitable for accurate impedance-based position measurements, a plurality of discrete, electrically-conducting elements are disposed either randomly or at predefined positions along the length of the sheath. The electrically-conducting elements pass through the sheath wall into the inner lumen of the sheath, and thus allow current to flow outward locally from the electrode at the catheter's distal tip to the body surface electrodes.

Typically, the electrically-conducting elements are small and isolated from one another. As such, current cannot flow along the sheath - Current can only traverse the sheath wall locally through the sheath wall via the elements. Adjacent elements are close enough to one another so as to allow current to traverse the wall locally near the position of the catheter tip in the sheath. As a result, the impedances or currents measured between the catheter electrode via the electrically-conducting elements and the body surface electrodes remain indicative of the catheter tip position.

In some embodiments, the sheath comprises an elongate electrically-insulating tube. Multiple holes are formed along the length of the insulating tube. The holes penetrate the outer wall, between the exterior of the tube and the internal lumen of the sheath. Multiple discrete, electrically conducting elements are then inserted into the respective holes to realize discrete localized electrically conducting paths through the sheath wall. In this manner, the sheath allows the impedance-based position sensing system to locate the distal tip of the catheter in the sheath in real time as the operator pushes the catheter through the lumen of the sheath.

The electrically conducting (typically metallic) elements may be implemented in various ways. In some embodiments, metal beads are mixed into insulating material, such as a polymer, which is pushed through a die to form the electrically transparent sheath by extrusion. In other embodiments, the polymer material is drawn over a tubular mold with openings, and metal is injected through the openings into the polymer to form an array of metal elements.

In yet other embodiments, holes are drilled into the tube and metal rivets are inserted into the holes so as to form the metal inserts along the length of the sheath. In other embodiments, springs are threaded through holes drilled in the tubing so as to form the metal inserts along the length of the sheath.

In some embodiments, the sheath comprises an internal layer that comprises a metallic braid, e.g., a braid of interwoven metallic filaments. In such embodiments, an insulating material may be disposed between the electrically conducting elements (e.g., the metal rivets or springs) and the braid, so as to prevent electrical shorting of the current traversing the metal element to the metal braid.

When using the disclosed sheaths, the operator can guide and maneuver the catheter through the sheath to the target region as the position of the distal tip is tracked within the sheath in real time. As a result, the electrically transparent sheath formed using the methods taught herein significantly enhances the control of the operator over the medical procedure.

### SYSTEM DESCRIPTION

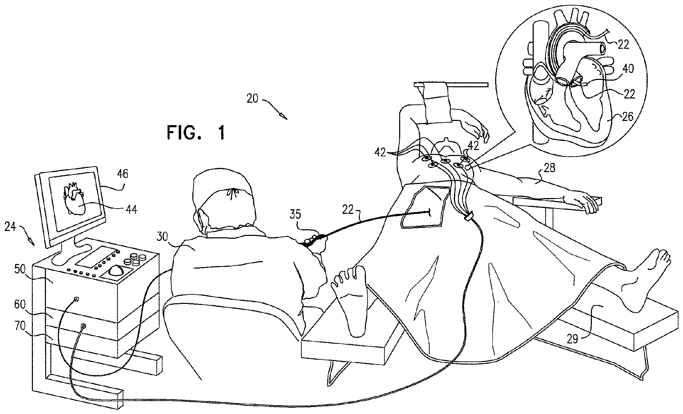

Fig. 1 is a schematic, pictorial illustration of a position sensing system 20, in accordance with an embodiment of the present invention. System 20 is used in determining the position a medical probe 22, typically a catheter. System 20 comprises a control console 24. Catheter 22 is percutaneously inserted through a suitable sheath into an internal body cavity, such as the chamber of a heart 26 in a living body 28 of a patient, lying on a gurney 29.

An operator 30 of the medical procedure manipulates catheter 22 in the vascular system of patient 28 with a hand 35 of operator 30, and guides a distal tip 40 of catheter 22 to the target region in the chamber of heart 26 to perform the medical procedure. Operator 30 can view an image 44 of the heart while monitoring the medical procedure on a display 46.

Control console 24 comprises a probe interface/control unit 50 for relaying signals to the at least one electrode disposed on the body of catheter 22 near distal tip 40. Usually the signals are relayed between the at least one electrode at distal tip 40 and probe interface/control unit 50 through multiple wires placed in the internal lumen of the catheter, which are not shown in Fig. 1 for sake of simplicity. Catheter 22 may also comprise additional electrodes and/or sensors placed at any suitable positions along the length of the catheter.

Additionally, a plurality of body surface electrodes 42 are fixed at suitable locations on the patient's body as shown in Fig. 1. Body surface electrodes 42 are connected by a cable to a body surface electrode interface/control unit 60 in control console 24.

For the exemplary embodiment shown in Fig. 1, position sensing system 20 locates the position of the at least one electrode disposed near the distal tip of catheter 20 in the body of patient 28 using real time impedance-based location tracking techniques. System 20 performs location tracking by driving a plurality of currents between the at least one electrode disposed near distal tip 40 of catheter 22 and multiple body electrode 42 fixed to the surface of the body of patient 28.

Control units 50 and 60 generate the plurality of currents and relay the measured currents to a processor 70. Processor 70 computes a plurality of respective impedances between the at least one electrode at the catheter distal tip and the plurality of the body surface electrode based on the measured currents. Processor 70 then computes the location of probe 22 in the patient's body based on the computed impedances. Processor 70 outputs the position of the catheter to operator 30 on display 46, which is typically visually mapped within image 44 of heart 26.

Probe 22 may comprise a catheter with one or more probe electrodes disposed near the distal tip. System 20 then computes a plurality of impedances between each of the multiple probe electrodes and the plurality of body surface electrodes at respective locations on the body of the patient.

As will be explained in detail below, the sheath used for guiding catheter 22 comprises multiple electrically-conducting elements (e.g., metal inserts) that allow electrical current to pass locally through the sheath wall. These elements enable system 20 to measure the position of the catheter distal tip using impedance measurements, even though the tube of the sheath is electrically insulating. In the present context, the term "metallic" means comprising metal so as to facilitate conduction of electrical current. A metallic element may be entirely made of metal, covered or plated with metal, or filled with metal, for example.

The position sensing system shown in Fig. 1 is depicted purely for conceptual clarity, and not by way of limitation of the embodiments of the present invention. Console 24 is configured to relay signals between the at least one electrode disposed near distal tip 40 of catheter 22 and the plurality of body electrodes 42. Console 24 also controls other components of system 20, so as to track the position of distal tip 40 of catheter 22 in heart 26. Body surface electrodes 42 may be placed in any suitable position on the patient's body so as to optimize the detection of the at least one electrode near the catheter's distal tip within the patient's body as described herein. In addition to position tracking, control console 24 may also be configured to perform therapeutic procedures such as RF ablation.

Any suitable parameters may be measured and/or processed by system 20, and output to a display 46. For example, a mapping of an image 44 of heart 26 with multiple local electro-cardiac signals measured by multiple electrodes near distal tip 40 of catheter 22 which contact multiple points in the heart cavity. The mapping may be output to display 46.

Processor 70 typically comprises a general-purpose computer, with suitable front end and interface circuits for transmitting and receiving signals from probe 22, and for controlling the other components of system 20 described herein. Processor 70 may be programmed in software to carry out the functions that are used by the system, and the processor may store data for the software in a memory. The software may be downloaded to console 24 in electronic form, over a network, for example, or it may be provided on non-transitory tangible media, such as optical, magnetic or electronic memory media. Alternatively, some or all of the functions of processor 70 may be carried out by dedicated or programmable digital hardware components.

### ELECTRICALLY TRANSPARENT SHEATHS FOR CATHETER POSITION SENSING SYSTEMS

In many catheter-based medical procedures, a sheath is first inserted into the vascular system of patient 28, until reaching the target region. The catheter itself is then guided through the sheath. Typically, the sheath is formed from lubricous polymer materials such as polypropylene, polyethylene, FEP (fluorinated ethylene propylene), and ETFE (ethylene tetrafluoroethylene), which are electrically insulating. Alternatively, however, the sheath may be fabricated from any other suitable material.

When using an electrically insulating sheath, current flow between the catheter electrodes and the body surface electrodes is blocked. As a result, the position of the distal tip of the catheter cannot be measured correctly by system 20 while in the sheath. In such a case, system 20 can detect and visualize the position of the catheter distal tip only when the distal tip emerges from the sheath. This obstruction slows and complicates the catheter guidance.

In the embodiments described herein, holes are formed in the sheath either randomly or at predefined locations along the length of the sheath. Electrically-conducting elements, e.g., metal inserts, are inserted into the holes. With the metal inserts in place, as the catheter moves within the sheath, electrical current is able to flow between the catheter tip electrode and the body surface electrodes, thus facilitating the impedance-based position measurements of system 20.

As a result, operator 30 can now quickly navigate the catheter through the lumen of the sheath into position at the target region since system 20 computes and displays the position of the sheath to the operator in real time. This feature significantly reduces the duration of the medical procedure.

### METHODS FOR FORMING ELECTRICALLY TRANSPARENT SHEATHS

In the embodiments presented herein, there are a number of methods for forming electrically transparent sheaths and particularly, the electrically-conducting elements. Several example implementations of electrically conducting elements are described below. Generally, however, these elements can be implemented in any other suitable way.

In a first embodiment, metal beads are mixed in the polymer material and the sheath is formed by extrusion. In a second embodiment, the polymer is drawn over a mold with openings along the length of the mold. Metal is injected through the openings into the polymer to form the metal elements in the sheath. In a third embodiment, holes are drilled into a multilayered polymer-based tube forming the sheath, and metal rivets are inserted into the holes to form the metal elements in the sheath. In a fourth embodiment, holes are drilled into a multilayered polymer-based tube forming the sheath, and spring coils are inserted through a hole formed in the tubing so as to form the metal elements in the sheath.

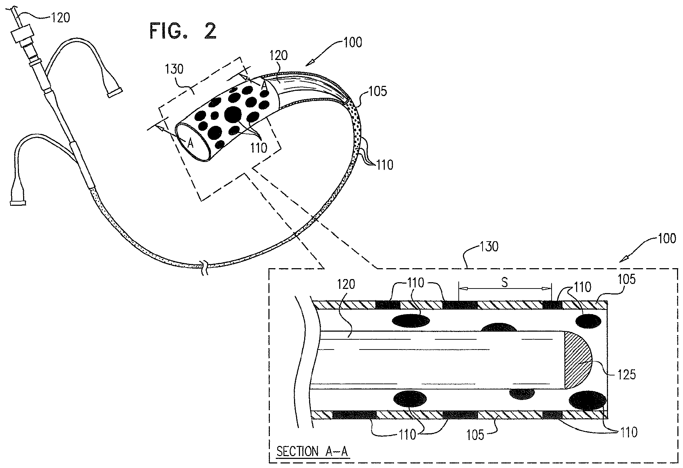

Fig. 2 is a schematic, pictorial illustration of a sheath 100 with metal filled holes 110, in accordance with an embodiment of the present invention. Multiple conducting holes 110 are typically filled with metal. Holes 110 are disposed along the length of sheath 100 with a spacing S between adjacent holes as shown in an inset 130 with a cross-sectional view of a catheter 120 with at least one electrode at a distal tip 125 in sheath 100. Sheath 100 comprises a layer 110, such as a polymer, such that the metal filled holes extend through the outer wall of sheath 100 to the inner lumen.

As electrode 125 disposed on the distal tip moves through sheath 100, current driven by system 20 through catheter electrode 125 is transmitted through the multiple metal holes typically closest to the distal tip, and through the body of patient 28 to the plurality of body surface electrodes 42, such that system 20 can detect the position of the catheter distal tip in sheath 100 based on the currents. Each of the multiple metal filled holes is electrically isolated one from another.

The metal filled holes are typically 1 mm in diameter with a 0.5 mm spacing between adjacent holes, but may be any suitable geometry. Alternatively, however, any other suitable hole dimensions and spacing can be used.

In some embodiments, metal filled holes 110 are arranged in an array with a fixed spacing S along the length of sheath 100. In other embodiments, metal filled holes 110 are arranged in an arbitrary or random fashion along the length of sheath with a random spacing S. The spacing S between adjacent metal filled holes can be oriented in any arbitrary direction along the length of the catheter. The spacing parameter S is used here for conceptual clarity, and not by way of limitation of the embodiments of the present invention.

The embodiment with multiple filled metal holes 110 arranged in a random fashion is shown in Fig. 2. Sheath 105 can be formed by mixing metallic beads, or any suitable metallic objects, into the polymer material, and using extrusion by pressing the polymer with the metallic bead composite through a die so as to construct sheath 100 with layer 105 with random metal filled holes 110 pattern shown in inset 130.

Similarly, the embodiment with multiple filled metal holes 110 arranged in a well-defined array pattern can be formed by drawing the polymer material over a tubular mold with openings in the mold arranged along the length of the mold with the desired well-defined array pattern. After the polymer is drawn over the mold, metal is injected through the openings and into the polymer, so as to form multiple filled metal holes 110 in the desired well-defined array pattern.

The embodiments described herein refer mainly to an electrode fitted at or near the distal end of catheter 22. Alternatively, however, the disclosed techniques can be used for measuring the positions of electrodes fitted at any desired position along the catheter. In some embodiments, an electrode may be fitted at a position along the catheter that is always inside the sheath. The disclosed techniques are especially valuable for locating such electrodes.

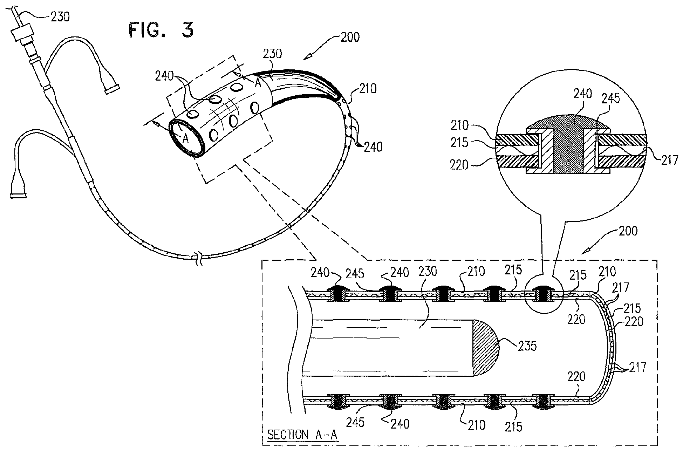

Fig. 3 is a schematic, pictorial illustration of a sheath 200 with holes filled with metal rivets 240, in accordance with an embodiment of the present invention. Multi-layered Sheath 200 comprises a an outer jacket layer 210, a metal braid layer 215 with metal braid filaments 217, and an inner liner layer 220 which lines the lumen of sheath 200. Examples of a multi-layered sheath by Teleflex Medical OEM can be found in the two specification documents entitled "Fluoropolymer-lined, Braid-Reinforced Catheter Shaft Design" and "Sterilization", which are incorporated herein by reference.

Holes are drilled through sheath 200 from the outer jacket into the sheath lumen at predefined locations along the length of the sheath. Metal rivets 240 are inserted into the drilled holes. The metal rivets are the metal elements needed to transmit the current to body surface electrodes 42 for detecting the position of a catheter 230 in sheath 200. The holes can be formed by any suitable method, such as drilling, and the metal rivets fastened in the holes.

However, after the holes are formed and metal rivets 240 inserted, the metal filaments 217 may electrically short the metal rivets to the metal braid over the length of the sheath in metal braid layer 215, which degrades position detection accuracy of the catheter's distal end. Position accuracy is maintained by using discrete current transmission points for detecting the position of catheter electrode 235 near the distal tip of catheter 230. To circumvent this, any suitable insulating material 245 is applied to the shaft of the metal rivet or to the sidewalls of the drilled holes so as to electrically isolate the metal rivets from the metal filaments in the metal braid layer.

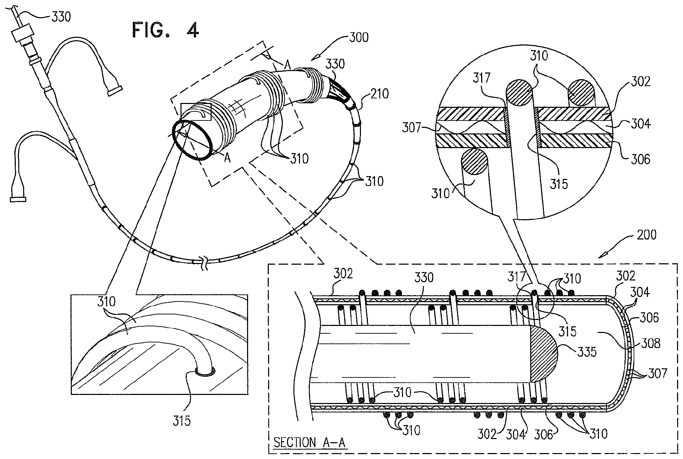

Fig. 4 is a schematic, pictorial illustration of a sheath 300 with holes filled with metal springs 310, in accordance with an embodiment of the present invention. Multi-layered Sheath 300 comprises a an outer jacket layer 302, a metal braid layer 304 with metal braid filaments 307, and an inner liner layer 306 which lines an inner lumen 308 of sheath 300.

Holes are drilled from the outer wall of sheath 300 into the sheath lumen at predefined locations along the length of the sheath. Metal springs 310 are then threaded through the drilled holes. The metal springs transmit the current to body surface electrode 42 for detecting the position of a catheter 330 in sheath 300. The holes can be formed by any suitable method. Typically, the metal springs threaded into the holes such that a portion of the spring coils remain wrapped around outer jacket 302, and a portion coil around inner liner layer 306 in inner lumen 308.

As explained above with reference to Fig. 3, after the holes are formed and metal springs 310 inserted, the metal filaments 307 may electrically short the metal rivets together in metal braid layer 304, which is deleterious for maintaining discrete current transmission points for detecting the position of catheter electrode 335 at the distal tip of catheter 330 accurately. To circumvent this, any suitable insulating material 317 is applied to the shaft of the metal spring or to the sidewalls of the drilled holes so as to electrically isolate the metal springs from the metal filaments in the metal braid layer.

In the embodiments shown in Figs. 2-4, the electrically conducting elements comprise a plurality of holes formed in the sheath filled with conducting materials, typically metal, metal rivets, or metal springs. The embodiments shown in Figures 2-4 are purely for conceptual clarity and not by way of limitations of the embodiments of the present invention. Any suitable method of filling the holes formed at predefined positions along the length of the sheath between the sheath outer wall to the sheath inner lumen with the electrically conducting elements may be used, which allow the transmission of an electrical signals (e.g., current and/or voltage) from the at least one electrode near the distal tip of the catheter to a plurality of body surface probes while inserted within the sheath.

### A METHOD FOR SENSING THE CATHETER POSITION IN THE LUMEN OF AN ELECTRICALLY TRANSPARENT SHEATH

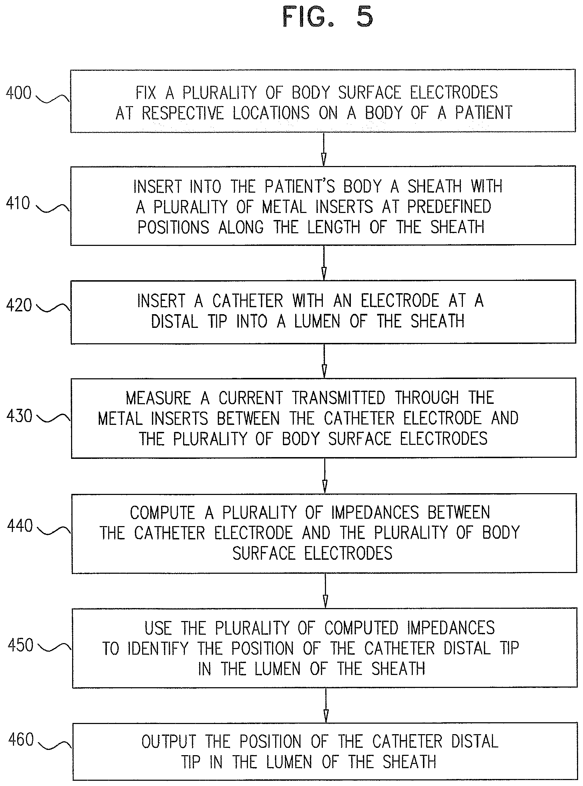

Fig. 5 is a flow chart that schematically illustrates a method for sensing the position of a catheter in an electrically-transparent sheath, in accordance with an embodiment of the present invention. In a fixing step 400, a plurality of body surface probes are fixed at respective locations on a body of a patient.

In an inserting step 410, a sheath with a plurality of electrically conducting elements (e.g., metal rivets or springs) distributed along the length of the sheath is inserted into the patient's body. In a second inserting step 420, a catheter with an electrode at a distal tip is inserted into a lumen of the sheath. In a measuring step 430, a current is measured that is transmitted through the electrically conducting elements between the catheter electrode and the plurality of body surface electrodes. In a computing step 440, a plurality of impedances is computed between the catheter electrode and the plurality of body surface electrodes. In a using step 450, the plurality of computed impedances is used to identify the position of the catheter distal tip in the lumen of the sheath. In an outputting step 460, the position of the catheter distal tip in the lumen of the sheath is outputted to the operator.

Although the embodiments described herein mainly address position sensing of catheters in an insulating sheath, the methods and systems described herein can also be used for location measurement of an intra-body probe in any suitable intra-body procedure.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

Aspects of the invention not yet claimed:
Aspect 1. A method, comprising:
   fixing a plurality of body surface electrodes at respective locations on a surface of a living body;
   inserting into the living body a sheath that comprises:
      an elongate tube, which comprises an outer wall that surrounds an inner lumen and has multiple holes along the tube penetrating the outer wall; and
      multiple electrically-conducting elements, which are inserted in the respective holes so as to allow transmission of electrical current between the inner lumen and an exterior of the outer wall;
   guiding through the inner lumen of the sheath a probe comprising at least one probe electrode;
   measuring the electrical current that flows via the elements between the at least one probe electrode and the body surface electrodes; and
   estimating a position of the at least one probe electrode based on the measured electrical current.
Aspect 2. The method according to aspect 1, wherein the metallic elements comprise one of:
   metallic beads filling the multiple holes;
   metallic rivets filling the multiple holes; and
   metallic springs threaded through the multiple holes.
Aspect 3. The method according to aspect 1, wherein the elongate tube of the sheath comprises metallic filaments forming a braid, and wherein the sheath further comprises an insulating material that insulates the elements from the metallic filaments of the braid.
Aspect 4. A method, comprising:
   producing an elongate tube, which comprises an outer wall that surrounds an inner lumen and has multiple holes along the tube penetrating the outer wall; and
   insertingmultiple electrically-conducting elements into the respective holes so as to allow transmission of electrical current between the inner lumen and an exterior of the outer wall.
Aspect 5. The method according to aspect 4, wherein inserting the electrically-conducting elements comprises i) inserting metallic beads into the respective holes, ii) inserting metallic rivets into the respective holes, or iii) threading metallic springs into the respective holes.
Aspect 6. The method according to aspect 4, wherein producing the tube and inserting the electrically-conducting elements comprise mixing metallic objects in an insulating material used for producing the tube, and then forming the tube by extrusion.
Aspect 7. The method according to aspect 4, wherein producing the tube comprises i) drawing insulating material over a mold having multiple openings, and wherein inserting the electrically-conducting elements comprises injecting metal through the openings, or ii) forming in the tube a braid of metallic filaments, and comprising applying an insulating material that insulates the elements from the metallic filaments of the braid.

## Claims

1. A sheath, comprising:
an elongate tube, which comprises an outer wall that surrounds an inner lumen and has multiple holes along the tube penetrating the outer wall; and
multiple electrically-conducting elements, which are inserted in the respective holes so as to allow transmission of electrical current between the inner lumen and an exterior of the outer wall.

2. The sheath according to claim 1, wherein the electrically-conducting elements comprise metallic beads filling the multiple holes.

3. The sheath according to claim 1, wherein the electrically-conducting elements comprise metallic rivets filling the multiple holes.

4. The sheath according to claim 1, wherein the electrically-conducting elements comprise metallic springs threaded through the multiple holes.

5. The sheath according to claim 1, wherein the elongate tube comprises metallic filaments forming a braid, and further comprising an insulating material that insulates the elements from the metallic filaments of the braid.

6. An apparatus, comprising:
a plurality of body surface electrodes, which are fixed at respective locations on a surface of a living body;
a sheath according to any of claims 1 to 5, which is inserted into the living body;
a probe, comprising at least one probe electrode, which is guided through the inner lumen of the sheath; and
a processor, which is configured to measure the electrical current that flows via the elements between the at least one probe electrode and the body surface electrodes, and to estimate a position of the at least one probe electrode based on the measured electrical current.
